# FASCICULE DE BREVET EUROPEEN

(11) **EP 0 641 225 B1**
(45) Date de publication et mention de la délivrance du brevet: **25.11.1998**
(21) Numéro de dépôt: 92918076.8
(22) Date de dépôt: 30.07.1992
(51) Int. Cl.: A61L 31/00, A61L 27/00

(54) **UTILISATION DE COLLAGENE RETICULE POUR LA FABRICATION D'UNE MEMBRANE SUTURABLE, BIOCOMPATIBLE A RESORPTION LENTE**
VERWENDUNG VON VERNETZTEM KOLLAGEN ZUR HERSTELLUNG EINER NÄHFÄHIGEN BIOVERTRÄGLICHEN LANGSAM RESORBIERBAREN MEMBRAN
UTILISATION OF CROSS-LINKED COLLAGEN FOR THE FABRICATION OF A STITCHABLE, BIOCOMPATIBLE, SLOW RESORPTION MEMBRANE

(30) Priorité: 02.08.1991 FR 9109909
(43) Date de publication de la demande: 08.03.1995
(73) Titulaire: COLETICA, 69007 Lyon (FR)
(72) Inventeur: ABDUL-MALAK, Nabil, F-69300 Caluire (FR); FOURCART, Jean, F-51170 Fismes (FR); HUC, Alain, F-69110 Ste-Foy-les-Lyon (FR)
(74) Mandataire: Portal, Gérard
(86) Numéro de dépôt international: FR9200750
(87) Numéro de publication internationale: WO9302718

(56) Documents cités:
- EP-A- 0 052 288
- EP-A- 0 156 740
- EP-A- 0 187 014
- EP-A- 0 331 786
- WO-A-90/12055
- WO-A-90/13302
- US-A- 4 280 954

## Description

L'invention concerne essentiellement l'utilisation de collagène réticulé par un agent de réticulation pour la fabrication d'une membrane suturable, biocompatible, à résorption lente, ainsi qu'une telle membrane. Une telle membrane peut être avantageusement utilisée pour la régénération tissulaire guidée.

Le concept de régénération tissulaire guidée a été récemment développé par Nieman et implique en pratique l'usage d'un matériau biocompatible capable de séparer in vivo deux populations cellulaires.

Le principe en est le suivant :
lorsqu'un espace vide est ménagé au sein d'un tissu vivant, celui-ci est comblé par la lignée cellulaire adjacente à ce vide la plus rapide à se multiplier, sauf si l'accès est volontairement limité à un unique type cellulaire qui alors colonisera seul le vide à combler.

Ce principe est mis à profit dans la régénération tissulaire guidée pour orienter la réparation des tissus lésés dans le sens souhaité par le clinicien.

Ainsi, par exemple, dans le cas de la parodontologie, il est très difficile d'obtenir une réparation du tissu ligamentaire périradiculaire lorsque celui-ci a été lésé. En effet, lors des processus de cicatrisation du parodonte, l'épithélium se régénère plus rapidement que le ligament et il a tendance à prendre la place de celui-ci.

La technique de régénération tissulaire guidée utilisée dans ce cas consiste à isoler la zone normalement occupée par le ligament, de façon à la rendre inaccessible à l'épithélium. Cette opération peut être réalisée à l'aide d'un matériau biocompatible implanté dans les tissus. La description d'un tel matériau fait l'objet de la présente invention.

A ce jour, deux types de produits sont connus pour être utilisés dans le but de cette régénération tissulaire guidée.

On connaît tout d'abord les membranes de polytétrafluoroéthytlène expansé commercialisées sous la marque Gore-Tex ^{R} , qui ne sont pas résorbables. Elles ont l'avantage de demeurer intactes tout au long du temps de leur implantation et remplissent ainsi parfaitement le rôle de barrière qu'elles ont à jouer.

Toutefois, elles ne peuvent pas être laissées en place indéfiniment et elles nécessitent deux interventions chirurgicales, une première pour poser le matériau, une seconde six à huit semaines plus tard, pour le déposer une fois le phénomène de régénération initié.

D'autre part, les membranes résorbables composées de collagène ou d'autres polymères comme les polyglycolates ne nécessitant pas d'intervention d'ablation car elles sont éliminées par résorption.

Par exemple, le document WO 90/13302 décrit la préparation de membranes biocompatibles, pour la régénération tissulaire, constitué notamment de collagène I et III non coagulé mais réticulé, à résorption lente, dont l'une des faces est imprégnée d'un principe actif.

D'autre part, le document EP-A-0 331 786 concerne aussi la préparation de membranes à base de collagène réticulé pour la formation d'implants ou le traitement de blessures, et notamment pour réaliser des hémostases (colonne 1, ligne 48 à colonne 2, ligne 11). Une régénération tissulaire guidée, en particulier pour la chirurgie dentaire, objet de l'invention, n'est pas décrite ou suggérée.

Encore, le document EP-A-0 050 288 concerne l'utilisation de collagène I réticulé pour la fabrication d'implants (page 2, lignes 3 à 18), mais une protéolyse est réalisée.

Le document EP-A-0 187 014 est relatif à la préparation de membranes biocompatibles à résorption lente à base de collagène réticulé et notamment d'atécollagène pour la régénération tissulaire guidée à base d'un gel de collagène qui est comprimé pour donner naissance à un réseau de fibres, ce gel pouvant être mélangé avec une suspension de fibres de collagène réticulé (voir les revendications et la description correspondante).

Le document US-A-4 280 954 est relatif à des matériaux composites, obtenus par réticulation d'une combinaison de collagène et d'un mucopolyssacharide, en particulier de chondroïtine-4-sulfate, pour la réalisation de sutures chirurgicales et de prothèses de biodégradabilité contrôlée. Une régénération tissulaire guidée en tant que telle n'est pas décrite dans ce document.

Le document WO-A-90/12055 est relatif à un procédé de réticulation du collagène par le diphylphosphorylazide et son utilisation comme biomatériau, mais une utilisation comme matériau de régénération tissulaire guidée n'est pas décrite ou suggérée.

Le document EP-A-0 156 740 est relatif à un procédé de fabrication de tubes à base de de collagène réticulé, comprenant une étape de coagulation du collagène, pour la fabrication de prothèses vasculaires et de sutures nerveuses, mais une utilisation pour la régénération tissulaire guidée n'est pas décrite, au contraire, il est recherché un moindre développement de cellules mésothéliales (page 7, lignes 27 à 29).

Cependant, leur temps de résorption est relativement court et elles ne permettent pas dans tous les cas d' initier une régénération suffisante car elles demeurent intactes trop peu de temps.

La présente invention a donc pour but de fournir un nouveau matériau de membrane de régénération tissulaire guidée qui soit suturable, biocompatible et à résorption lente.

La présente invention a encore pour but de fournir un procédé particulier de fabrication du matériau de base de telles membranes.

La présente invention permet de résoudre ce problème technique pour la première fois, d'une manière simple, fiable, peu coûteuse, utilisable à l'échelle industrielle et médicale.

Ainsi, selon un premier aspect, la présente invention fournit une utilisation de collagène réticulé par un agent de réticulation, pour la fabrication d'une membrane de préférence de régénération tissulaire guidée suturable, biocompatible à résorption lente, caractérisée en ce que ladite membrane comprend soit du collagène réticulé à partir d'un collagène de départ à l'état coagulé par suite d'une coagulation d'un gel de collagène par un agent coagulant, soit se présente sous forme d'une membrane mixte comprenant une éponge d'un mélange collagène ou atélocollagène-glycosaminoglycanne sur laquelle a été coulé un gel de collagène avant réticulation de l'ensemble, soit comprend une éponge d'un mélange de collagène et de chondroïtine-4-sulfate qui a été comprimée sous une pression de 150 bars.

Selon une variante de réalisation, le collagenè de départ et à l'état coagulé par suite d'une coagulation d'un gel de collagène avant réticulation de l'ensemble.

Selon une variante de réalisation, le taux de réticulation est tel qu'il apparaît une augmentation d'au moins 15°C dans la température de dénaturation du collagène par rapport au collagène natif, et encore mieux d'au moins 20°C.

L'agent de réticulation peut être choisi parmi tout agent de réticulation de collagène connu. Il peut par exemple s'agir d'une aldéhyde comme une dialdéhyde, en particulier la glutaraldéhyde. Mais, de préférence, l'agent de réticulation est constitué par le diphénylphosphorylazide (en abrégé DPPA). Contrairement à la plupart des autres agents réticulants, le DPPA induit la réticulation et ne se fixe pas sur le matériau.

Le procédé de réticulation lui-même est bien connu à l'homme de l'art. Pour un procédé de réticulation au DPPA, on peut se reporter à une demande antérieure du déposant publiée sous le n° FR-A-2 645 870.

Comme collagène, on peut utiliser du collagène natif, en particulier de type I ou de type III.

Selon une variante de réalisation particulière, on peut aussi utiliser de l'atélocollagène, bien que cela soit moins préféré.

Selon une autre variante de réalisation avantageuse, la membrance mixte précitée est réalisée poreuse en ayant ainsi l'aspect d'une éponge en ayant été préparée par une étape de lyophilisation d'un gel de départ formé par un mélange collagène ou atélocollagène-glycosaminoglycanne.

Selon un deuxième aspect, la présente invention couvre également une membrane de préférence de génération tissulaire guidée suturable, peu compatible à résorption lente, comprenant du collagène réticulé par un agent de réticulation, caractérisée en ce que ladite membrane comprend soit un collagène réticulée à partir d'un collagène de départ à l'état coagulé par suite d'une coagulation d'un gel de collagène par un agent coagulant, soit se présente sous forme d'une membrane mixte comprenant une éponge d'un mélange collagène ou atélocollagène-glycosaminoglycanne sur laquelle a été coulé un gel de collagène avant réticulation de l'ensemble, soit comprend une éponge d'un mélange de collagène et de chondroïtine-4-sulfate qui a été comprimée sous une pression de 150 bars.

Selon une variante de réalisation, le taux de réticulation est prévu pour obtenir une augmentation d'au moins 15°C dans la température de dénaturation du collagène réticulé par rapport au collagène natif, encore mieux d'au moins 20°C. L'agent de réticulation préféré est le diphénylphosphorylazide, en abrégé DPPA.

Selon une variante de réalisation, le collagène est de l'atélocollagène.

Selon une variante de réalisation avantageuse, le collagène est un collagène natif de type I ou de type III.

Selon une autre variante de réalisation, le collagène ou l'atélocollagène est mélangé à un glycosaminoglycanne avant réticulation de l'ensemble par l'agent de réticulation précité.

Selon une autre variante de réalisation de la membrane mixte précitée, celle-ci est poreuse notamment en ayant été préparée par une étape de lyophilisation à partir d'un gel de départ d'un mélange collagène ou atélocollagène-glycosaminoglycanne.

Selon une autre variante de réalisation, cette membrane mixte comprend une éponge réalisée à partir d'un mélange collagène natif et d'un glycosaminoglycanne, en particulier le chondroïne-4-sulfate, sur laquelle est coulé un gel de collagène natif. Avantageusement, l'éponge a été comprimée sous pression, en particulier sous une pression de 150 bars avant coulage du gel.

Comme glycosaminoglycannes, dans le cadre de la présente invention, on peut utiliser avantageusement un glycosaminoglycanne de structure, en particulier l'acide hyaluronique, le chondroïtine-4-sulfate, le chondroïtine-6-sulfate, le dermatane-sulfate, l'héparane-sulfate, le kératane-sulfate, l'héparine et ses dérivés, en particulier les héparines de bas poids moléculaire compris entre 2 000 et 10 000.

La proportion relative de glycosaminoglycannes relativement au collagène ou à l'atélocollagène est de préférence comprise entre 18 et 25%.

Le mélange du glycosaminoglycanne avec le collagène ou l'atélocollagène a lieu sous forme de solution. Par exemple, le glycosaminoglycanne est sous forme d'une solution aqueuse de glycosaminoglycannes contenant de 0,5 à 4 % en poids, plus particulièrement de 0,5 à 2 % en poids et encore de préférence environ 1 % de glycosaminoglycannes. De même, le collagène ou l'atélocollagène peut être sous forme d'une solution aqueuse ayant une concentration entre 0,5 et 2 % en poids et encore de préférence environ 1 % en poids de collagène ou d'atélocollagène. La solution de collagène ou d'atélocollagène peut être préparée selon l'invention par dissolution de fibres de collagène ou d'atélocollagène dans une solution aqueuse légèrement acide. En particulier, il peut s'gir d'une solution aqueuse d'acide acétique 0,1 M.

On peut prévoir d'amener le mélange de collagène, ou d'atélocollagène, et de glycosaminoglycannes à un pH voisin de la neutralité et en particulier à un pH compris entre 6,5 et 8. On peut utiliser à cet effet une solution aqueuse d'hydroxyde de sodium.

Dans le cadre de l'invention, on peut préparer une membrane à partir d'un collagène de départ à l'état coagulé par un agent coagulant.

Ce procédé comprend la préparation d'un gel de collagène caractérisé en ce qu'on réalise une coagulation de ce gel par un agent coagulant comprenant une solution ammoniacale ayant de préférence un effet déshydratant.

Selon un mode de réalisation avantageux de ce procédé de coagulation, la solution ammoniacale est une solution ammoniacale organique utilisant de l'acétone comme agent déshydratant. On observe en effet un effet de synergie par la combinaison acétone/ammoniaque pour la coagulation du gel et pour l'élimination de l'eau présente dans le gel.

On préfère une proportion relative acétone/ammoniaque comprise entre 50/50 et 80/20 en volume, une proportion volumique encore particulièrement préférée est 70/30 d'acétone/ammoniaque.

Selon une variante de réalisation avantageuse, lorsque les quantités de gel à coaguler sont relativement importantes, on réalise un renouvellement de la solution coagulante en cours de coagulation.

Selon une variante de réalisation particulière, le gel est coulé à travers une filière de section et de forme appropriée pour obtenir un gel coagulé de forme appropriée. Lorsque la section de la filière est rectangulaire, on obtient ainsi un film qui par la suite va constituer la membrane.

Le gel coagulé obtenu peut ensuite être soumis à une réticulation par l'agent de réticulation précité.

L'invention comprend encore un procédé de fabrication d'une membrane mixte de préférence de régénération tissulaire guidée suturable, biocompatible à résorption lente, comprenant du collagène réticulé, caractérisé en ce qu'on prépare tout d'abord une éponge de collagène sur laquelle on coule ensuite un gel de collagène et on réticule l'ensemble par un agent de réticulation, en particulier le diphénylphosphorylazide. Avantageusement, l'éponge du collagène comprend un mélange de collagène et de mucopolysaccharide, en particulier de chondroïtine-4-sulfate.

D'autres variantes de réalisation du procédé apparaissent également en relation avec les aspects précédents de l'invention. En particulier, le collagène peut être du collagène natif, en particulier de type I ou de type III, ou encore être en pratique de l'atélocollagène, c'est-à-dire du collagène débarrassé de ses télopeptides. En outre, le collagène peut être mélangé avec un glycosaminoglycanne.

On comprend ainsi que l'invention permet d'aboutir à tous les avantages techniques déterminants précédemment énoncés ainsi qu'aux avantages techniques qui apparaîtront à l'homme de l'art à partir de la description explicative suivante de l'invention faite en référence à deux modes de réalisation actuellement préfèrés de Dans les exemples, les pourcentages sont donnés en poids, sauf indication contraire.

### Exemple 1

### Préparation d'une membrane de collagène simple réticulé

### A - Extraction du collagène natif et préparation du gel :

Un gel est préparé à partir de peaux de veaux préalablement lavées et épilées par un mélange de chaux-sulfure (chaux : 4 %, sulfure de sodium : 3 %).

Elle est ensuite déchaulée dans un bain contenant du chlorure d'ammonium (2 %) et du métalisulfite de sodum (0,5 %). Elle est ensuite neutralisée, puis les sels sont éliminés par deux lavages à l'eau. Elle est alors broyée, puis lavée par du tampon phosphate pH 7,8 (dihydrogénophosphate de potassium 0,78 g/l et monohydrogénophosphate disodique 21,7 g/l). Le phosphate est ensuite éliminé par deux lavages successifs à l'eau permutée.

Le broyat est alors acidifié par une solution d'acide acétique à 10 %, la quantité d'acide acétique étant de 5 % par rapport au collagène. Le broyat est alors malaxé afin d'obtenir une pâte homogène. Cette pâte est ensuite diluée pour obtenir un gel ayant une concentration de 0,75 % en collagène natif.

### B - Préparation du film

Le gel obtenu est dégazé sous vide puis coulé dans un bain coagulant à travers une filière rectangulaire dont la section a une hauteur de 0,5 mm. La solution coagulante est un mélange acétone/ammoniaque (V/V 70/30) qui est renouvelé tous les 250 ml de gel.

Le film obtenu est alors séché à l'air à température ambiante sur un support plastique en polytétrafluoroéthylène. Une fois sec, le film est aisément dècollé de son support.

### C - Réticulation du film

Le film est alors placé pendant 24 h à 4°C dans une solution de diméthylformamide (DMF) contenant 0,5 % de diphènylphosphorylazide (DPPA), la concentration étant exprimée en volume. Le DPPA est éliminé de la membrane par rinçage dans une solution de tampon borate pH 8,9 (tétraborate de sodium 0,04 M, acide borique 0,04 M).

La membrane est alors incubèe pendant 15 h dans la solution tampon borate pH 8,9, puis rincée 5 fois à l'eau désionisée avant d'être placée dans une solution de glycérol à 10 %.

Elle est alors séchée à l'air et stérilisée par radiation γ à une dose de 25 kGy (kilogray). Les températures de début et de fin de dénaturation du collagène de cette membrane sot respectivement 64 et 80°C.

### Exemple 2

### Préparation d'une membrane mixte de collagène glycosaminoglycanne réticulé

### A - Extraction du collagène natif et préparation du gel

Un gel est préparé à partir de peaux de veaux préalablement lavées et épilées par un mélange de chaux-sulfure (chaux : 4 %, sulfure de sodium : 3 %).

Elle est ensuite déchaulée dans un bain contenant du chlorure d'ammonium (2 %) et du métalisulfite de sodum (0,5 %). Elle est ensuite neutralisée, puis les sels sont éliminés par deux lavages à l'eau. Elle est alors broyée, puis lavée par du tampon phosphate pH 7,8 (dihydrogénophosphate de potassium 0,78 g/l et monohydrogénophosphate disodique 21,7 g/l). Le phosphate est ensuite éliminé par deux lavages successifs à l'eau permutée.

Le broyat est alors acidifié par une solution d'acide acétique à 10 %, la quantité d'acide acétique étant de 5 % par rapport au collagène. Le broyat est alors malaxé afin d'obtenir une pâte homogène. Cette pâte est ensuite diluée pour obtenir un gel ayant une concentration de 0,75 % en collagène natif.

### B - Préparation du chondroïtine-4-sulfate

Des cloisons nasales d'agneau débarrassées des tissus musculaires et adipeux sont hachées et broyées par extrusion à travers une grille comportant des trous de 4 mm ; le broyat est placé pendant 24 h à une température de 6°C dans un tampon de chlorure de potassium (11,8 g/L de KCL, 78,8 mg/l de cystéine, ETDA 180 mg/l) renfermant 1 % de papaïne "MERCK". La proportion étant de 130 g de broyat pour 1 l de tampon.

Le surnageant est séparé du culot par centrifugation en continu à l'aide d'une décanteuse tournant à 4 000 tr/min. Au surnageant, sont alors ajoutés 40 g/l d'acide trichloracétique. Le précipité est éliminé par centrifugation en continu selon la technique précédente. Le surnageant est neutralisé à l'aide de soude en pastille. Le mélange est alors dialysé contre de l'eau désionisée et stérile à l'aide de boyaux dont le seuil de coupure est compris entre 6 000 et 8 000 daltons. La solution dialysèe est lyophilisée. Le chondroïtine-4-sulfate est obtenu à l'état sec.

### C - Préparation de l'éponge collagène chondroïtine-4-sulfate

A 1 l de gel à 0,75 % de collagène, sont ajoutés 1,87 g de chondroïtine-4-sulfate. Après neutralisation, le mélange est agité puis lyophilisé. L'éponge obtenue est pressée pendant 15 s sous une pression de 150 bars.

### D - Préparation de la membrane mixte

Le gel de collagène à 1 % est coulé sur l'éponge comprimée à l'aide d'une filière dont la section a une hauteur de 0,3 cm. 10 ml de gel sont déposés sur 35 cm² d'éponge. La membrane ainsi réalisée est séchée à l'air libre.

### E - Réticulation chimique de la membrane

La membrane séchée est incubée pendant 24 h à 4°C dans une solution de DMF contenant 0,5 % de DPPA, la concentration étant exprimée en volume. Le DPPA est éliminé de la membrane par rinçage dans une solution de tampon borate pH 8,9 (tètraborate de sodium 0,04 M, acide borique 0,04 M). La membrane est alors incubée pendant 15 h dans la solution tampon borate pH 8,9, puis rincée 5 fois à l'eau désionisée avant d'être placée dans une solution aqueuse de glycérol à 10 %.

La membrane est ensuite sèchée à l'air et stérilisée par radiation γ à une dose de 25 kGy (kilogray). Les températures de début et de fin de dénaturation du collagène de cette membrane sont respectivement 60 et 85°C.

Les membranes selon l'invention peuvent être utilisées comme un matériau de régénération tissulaire guidé, de préférence en chirurgie dentaire pour réaliser par exemple le comblement des poches parondontales, le réhaussement des crêtes maxillo-mandibulaires, la régénération osseuse périimplantaire.

Les poches parondontales sont des pertes osseuses dentaires périradiculaires secondaires à une attaque bactérienne des tissus de soutien de la dent.

Elles sont caractérisèes par une perte de la substance osseuse normalement présente autour de la racine de la dent et qui a pour mission de la soutenir sur la mâchoire.

Dans ce cas, pour utiliser la membrane de collagène, le praticien réalise un lambeau d'épaisseur totale pour exposer la lésion osseuse. Il applique la membrane sur l'os de façon à recouvrir complètement le défaut et à avoir un léger dépassement coronaire. Il referme enfin le lambeau en le suturant de façon à laisser dépasser très légèrement la membrane dans le sulcus.

L'intervention peut être également réalisée avec l'emploi concommitant de biomatériaux et comblement osseux. Cette technique permet une réparation des tissus lésés en 4 à 8 mois.

L'édentation d'une zone buccale s'accompagne souvent de pertes osseuses importantes. Le chirurgien peut récupérer ces pertes en employant la membrane sur l'os de façon à recouvrir et déborder la perte, en prenant soin de donner à l'espace à reconstruire entre la membrane et l'os la forme souhaitée pour la reconstruction et au besoin de maintenir cette forme par l'emploi sousjacent à la membrane d'un matériau compatible avec la repousse osseuse. Il referme enfin soigneusement le lambeau pour obtenir une reconstruction en 4 à 8 mois.

L'implantation chirurgicale de racines métalliques artificielles biocompatibles ou implants dentaires dans l'os des mâchoires édentées est une technique largement utilisée en chirurgie dentaire.

Il arrive souvent que ces implants soient posés dans des conditions qui ne leur permettent pas d'être en contact avec l'os sur toute leur surface radiculaire, cet os manquant à certains endroits.

Cette fois encore, l'emploi de membranes de collagène va permettre au chirurgien de réparer le défaut osseux contigu à l'implant.

Il lui suffira dans ce cas de recouvrir la zone osseuse où est posé l'implant avec une membrane, avant de refermer le lambeau levé en début d'intervention pour obtenir une parfaite ostéintégration de l'implant en 3 à 6 mois.

La présente invention couvre tous les moyens consistant en des équivalents techniques des moyens décrits ainsi que leurs diverses combinaisons.

Par ailleurs, l'invention couvre toute caractéristique qui apparaît être nouvelle relativement à un état de la technique quelconque, qui résulte de la description précédente prise dans son ensemble.

## Revendications

1. Membrane de préférence de régénération tissulaire guidée suturable, biocompatible à résorption lente, comprenant du collagéne réticulé par un agent de réticulation caracterisée en ce que ladite membrane comprend soit un collagène réticulé à partir dut collagéne de départ a l'état coagulé par suite d'une coagulation d'un gel de collagéne par un agent coagulant, soit se présente sous forme d'une membrane mixte comprenant une éponge d'un mélange collagéne ou atélocollagéne-glycosaminoglycanne sur laquelle a été coulé un gel de collagéne avant réticulation de l'ensemble, soit comprend une éponge d'un mélange de collagène et de chondroïtine---sulfate qui a été comprimée sous une pression de 150 bars.

2. Membrane selon la revendication 1,caracterisée en ce qu'il s'agit de collagène natif en particulier de type I ou de type III.

3. Membrane selon la revendication 1, charactérisée en ce qu'il s'agit d'atélocollagéne.

4. Membrane selon l'une des revendication 1 à 3, caractérisée et ce que le collagène ou l'atélocollagéne est mélangé à un glycosaminoglycanne.

5. Membrane selon l'une des revendications 1 à 4, caractérisée en ce que le taux de réticulation est prévu pour obtenir une augmentation d'au moins 15°C dans la température de dénaturation du collagène réticulé par rapport au collagène natif, et encore mieux d'au moins 20°C.

6. Membrane selon l'une des revendications 1 à 5, caracterisée en ce que l'agent de réticulation est le diphénylphosphorylazide.

7. Membrane selon l'une des revendications 1 à 6, caracterisée en ce que'il s'agit d'une membrane mixte comprenant une éponge réalisée à partir d'un mélange de collagéne natif et d'un glycosaminoglycanne. en particulier le chondroïtine-4-sulfate, sur laquelle est coulé un gel de collagéne natif.

8. Membrane selon la revendication 7, caracterisée en ce que l'eponge a été comprimée sous pression, en particulier sous une pression de 150 bars avant coulage du gel.

9. Membrane selon l'une des revendications 1 à 8, caracterisée en ce que la proportion relative de glycosaminoglycanne relativement au collagéne ou à l'atélocollagéne est comprise entre 18 et 25%.

10. Membrane selon l'une des revendications 1 à 9, caractérisée en ce que le glycosaminoglycanne est un glycosaminoglycanne de structure, en particulier l'acide hyaluronique, le chondroïtine-4-sulfate, le chondroïtine-6-sulfate, le dermatane-sulfate, l'héparane-sulfate, le kératane-sulfate, l'héparine et ses dérivés, en particulier les héparines de bas poids moléculaire compris entre 2 000 et 10 000.

11. Membrane selon l'une des revendications 1 à 10, caractérisée en ce que le mélange de collagène ou d'atécollagéne, et de glycosaminoglycanne, est obtenu à partir d'une solution aqueuse de glycosaminoglycanne contenant de 0,5 à 4 % en poids de glycosaminoglycanne et d'une solution aqueuse de collagène ou d'atélocollagéne ayant une concentration entre 0,5 et 2 % en poids de collagène ou d'atélocollagène, qui est avantageusement amenée à un pH voisin de la neutralité, en particulier un pH compris entre 6,5 et 8.

12. Procédé de fabrication d'une membrane mixte de préférence de régénération tissulaire guidée suturable, biocompatible à résorption lente, comprenant du collagène réticulé, caractérisé en ce qu'on prépare tout d'abord une éponge d'un mélange collagène ou atélocollagéne-glycosaminoglycanne, sur laquelle on coule ensuite un gel de collagéne et on réticule l'ensemble par un agent de réticulation, en particulier le diphénylphosphorylazide.

13. Procédé selon la revendication 12, caractérisé en ce que l'éponge de collagène comprend un mélange de collagène natif et d'un glycosaminoglycanne, en particulier de chondroïtine-4-sulfate.

14. Procédé selon la revendication 12 ou 13, caractérisé en ce qu'on prépare une éponge d'un mélange de collagéne et de chondroïtine-4-sulfate que l'on comprime sous une pression de 150 bars avant coulage du gel de collagéne.

15. Utilisation de collagène réticulé par un agent de réticulation, pour la fabrication d'une membrane de préférence de régénération tissulaire guidée suturable, biocompatible à résorption lente, caractérisée en ce que ladite membrane comprend soit du collagène réticulé à partir d'un collagéne de départ à l'état coagulé par suite d'une coagulation d'un gel de collagéne par un agent coagulant, soit se présente sous forme d'une membrane mixte comprenant une éponge d'un mélange collagéne ou atélcollagene-glycosaminoglycanne sur laquelle a été coulé un gel de collagéne avant réticulation de l'ensemble, soit comprend une éponge d'un mélange de collagène et de chondroïtine-4-sulfate qui a été comprimée sous une pression de 150 bars.

16. Utilisation selon la revendication 15, caractérisée en ce que la membrane est telle que définie dans l'une quelconque des revendications 2 à 11.

17. Utilisation selon la revendication 15 ou 16, caractérisée en ce que la membrane est utilisée comme un matériau de régénération tissulaire guidée en chirurgie dentaire pour réaliser par exemple le comblement des poches parondontales, le réhaussement des crêtes maxillo-mandibulaires, la régénération osseuse périimplantaire.

## Claims

1. A membrane, preferably a suturable membrane for guided tissue regeneration, biocompatible slow-resorbing, comprising a crosslinked collagen with a crosslinking agent, characterised in that said membrane comprises either a collagen crosslinked from a coagulated native collagen after coagulation of a gel of collagen by a coagulating agent, or is present in the form of a mixed membrane comprising a sponge of a collagen or atelocollagen-glycosaminoglycan mixture on which a gel of collagen has been deposited before crosslinking the whole, or comprising a sponge of a collagen and chondroïtine-4-sulfate mixture which has been compressed under a pressure of 150 bars.

2. Membrane according to claim 1, characterized in that it is a native collagen, particularly of type I or type III.

3. Membrane according to claim 1, characterized in that it is atelocollagen.

4. Membrane according to claims 1 to 3, characterized in that the collagen or atelocollagen is mixed with a glycosaminoglycan.

5. Membrane according to claims 1 to 4, characterized in that the degree of crosslinking is such as to increase the denaturation temperature of the crosslinked collagen by at least 15°C, preferably at least 20°C, compared with native collagen.

6. Membrane according to claims 1 to 5, characterized in that the crosslinking agent is diphenylphosphorylazide.

7. Membrane according to claims 1 to 6, characterized in that the mixed membrane contains a sponge realized from a mixture of native collagen and of a glycosaminoglycan, in particular chondroitin-4-sulfate, on which has been deposited a native collagen.

8. Membrane according to claims 7, characterized in that the sponge is a compressed sponge, in particular under a pressure of 150 bars, before the gel is run.

9. Membrane according to any one of claims 1 to 8, characterized in that the proportion of glycosaminoglycan relative to the collagen or atelocollagen is comprised between 18 and 25%.

10. Membrane according to any one of claims 1 to 9, characterized in that glycosaminoglycan is a structural glycosaminoglycan, in particular hyaluronic acid, chondroitin-4-sulfate, chondroitin-6-sulfate, dermatan-sulfate, heparan-sulfate, keratan-sulfate, heparin and its derivatives, in particular heparins with a low molecular weight of between about 2000 and about 10,000.

11. Membrane according to any one of the claims 1 to 10, characterized in that the mixture of collagen or atelocollagen and of glycosaminoglycan is in the form of an aqueous solution of glycosaminoglycan containing from 0,5 to 4% by weight of glycosaminoglycan and of an aqueous solution of collagen or atelocollagen having a concentration of between 0,5 and 2% by weight of collagen or atelocollagen, which are advantageously brought to a pH close to neutrality and in particular to a pH of between 6.5 and 8.

12. Process of manufacture of a mixed membrane, preferably a suturable membrane for guided tissue regeneration, biocopmatible slow-resorbing, comprising a crosslinked collagen, characterized in that it is first prepared a sponge of mixed collagen or atelocollagen-glycosaminglycan, on which is then run a collagen gel, and the whole is crosslinked with a crosslinking agent, in particular diphenylphosporylazide.

13. Process according to claim 12, characterized in that the collagen sponge comprises a mixture of native collagen and of a glycosaminoglycan, in particular chondroitin-4-sulfate.

14. Process of claim 12 or 13, characterized in that a sponge of a collagen and chondroitin-4-sulfate mixture is prepared, compressed under a pressure of 150 bars, before running the gel of collagen.

15. Use of crosslinked collagen with a crosslinking agent, for the manufacture of a membrane, preferably a suturable membrane for guided tissue regeneration, biocompatible slow-resorbing, characterised in that said membrane comprises either a collagen crosslinked from a coagulated native collagen after coagulation of a gel of collagen by a coagulating agent, or is present in the form of a mixed membrane comprising a sponge of a mixture of collagen or atelocollagen-glycosaminoglycan on which a collagen gel has been run before crosslinking the whole, or comprising a sponge of a mixture of collagen and of chondroitin-4-sulfate which has been compressed under a pressure of 150 bars.

16. Use according to claim 15, characterized in that said membrane is as defined in any one of claims 2 to 11.

17. Use of claim 15 or 16, characterized in that said membrane is used as a material for guided tissue regeneration in dental surgery, for example for filling periodontal pockets, raising maxillo-mandibular ridges or for regenerating bone around an implant.

## Patentansprüche

1. Als Nahtmaterial verwendbare, biokompatible Membran mit langsamer Resorption, vorzugsweise zur gezielten Geweberegeneration, welche mit einem Vernetzungsmittel vernetztes Kollagen umfaßt, dadurch gekennzeichnet, daß die Membran entweder ein vernetztes Kollagen, ausgehend von einem Ausgangskollagen im koagulierten Zustand nach einer Koagulation eines Kollagengels mit einem Koagulierungsmittel, umfaßt, oder in Form einer Mischmembran vorliegt, die einen Schwamm einer Kollagen- oder Atelokollagen-Glykosaminoglykan-Mischung umfaßt, auf den ein Kollagengel vor der Vernetzung des Ganzen gegossen wurde, oder einen Schwamm einer Kollagen- und Chondroitin-4-sulfat-Mischung umfaßt, der unter einem Druck von 150 bar komprimiert wurde.

2. Membran nach Anspruch 1, dadurch gekennzeichnet, daß es sich um natives Kollagen, insbesondere vom Typ I oder vom Typ III, handelt.

3. Membran nach Anspruch 1, dadurch gekennzeichnet, daß es sich um Atelokollagen handelt.

4. Membran nach einem der Ansprüche 1 bis 3, dadurch gekennzeichnet, daß das Kollagen oder das Atelokollagen mit einem Glykosaminoglykan gemischt ist.

5. Membran nach einem der Ansprüche 1 bis 4, dadurch gekennzeichnet, daß der Vernetzungsgrad so vorgesehen ist, daß eine Erhöhung der Denaturierungstemperatur des vernetzten Kollagens, bezogen auf das native Kollagen, von zumindest 15°C oder bevorzugter zumindest 20°C erhalten wird.

6. Membran nach einem der Ansprüche 1 bis 5, dadurch gekennzeichnet, daß das Vernetzungsmittel Diphenylphosphorylazid ist.

7. Membran nach einem der Ansprüche 1 bis 6, dadurch gekennzeichnet, daß es sich um eine Mischmembran handelt, die einen Schwamm umfaßt, der ausgehend von einer Mischung von nativem Kollagen und einem Glykosaminoglykan, insbesondere Chondroitin-4-sulfat, hergestellt ist, und auf den ein natives Kollagengel gegossen wird.

8. Membran nach Anspruch 7, dadurch gekennzeichnet, daß der Schwamm vor dem Aufgießen des Gels unter einem Druck, insbesondere unter einem Druck von 150 bar, komprimiert wurde.

9. Membran nach einem der Ansprüche 1 bis 8, dadurch gekennzeichnet, daß die relative Menge an Glykosaminoglykan, bezogen auf das Kollagen oder auf das Atelokollagen, zwischen 18 und 25 % beträgt.

10. Membran nach einem der Ansprüche 1 bis 9, dadurch gekennzeichnet, daß das Glykosaminoglykan ein Struktur-Glykosaminoglykan ist, insbesondere Hyaluronsäure, Chondroitin-4-sulfat, Chondroitin-6-sulfat, Dermatansulfat, Heparansulfat, Keratansulfat, Heparin und seine Derivate, insbesondere Heparine mit einer niedrigen Molmasse zwischen 2000 und etwa 10 000.

11. Membran nach einem der Ansprüche 1 bis 10, dadurch gekennzeichnet, daß die Mischung von Kollagen oder Atelokollagen und Glykosaminoglykan erhalten wird, ausgehend von einer wässerigen Glykosaminoglykan-Lösung, die 0,5 bis 4 Masse-% Glykosaminoglykan enthält, und einer wässerigen Kollagen- oder Atelokollagen-Lösung, die eine Konzentration zwischen 0,5 und 2 Masse-% Kollagen oder Atelokollagen aufweist, und die vorteilhaft auf einen pH nahe bei der Neutralität, insbesondere einen pH zwischen 6,5 und 8, geführt wird.

12. Verfahren zur Herstellung einer als Nahtmaterial verwendbaren, biokompatiblen Mischmembran mit langsamer Resorption, vorzugsweise zur gezielten Geweberegeneration, dadurch gekennzeichnet, daß zuerst ein Schwamm einer Kollagen- oder Atelokollagen-Glykosaminoglykan-Mischung hergestellt wird, auf den dann ein Kollagengel gegossen wird, und das Ganze mit einem Vernetzungsmittel, insbesondere Diphenylphosphorylazid, vernetzt wird.

13. Verfahren nach Anspruch 12, dadurch gekennzeichnet, daß der Kollagenschwamm eine Mischung von nativem Kollagen und einem Glykosaminoglykan, insbesondere Chondroitin-4-sulfat, umfaßt.

14. Verfahren nach Anspruch 12 oder 13, dadurch gekennzeichnet, daß ein Schwamm einer Mischung von Kollagen und Chondroitin-4-sulfat hergestellt wird, der vor dem Aufgießen des Kollagengels unter einem Druck von 150 bar komprimiert wird.

15. Verwendung von mit einem Vernetzungsmittel vernetztem Kollagen bei der Herstellung einer als Nahtmaterial verwendbaren, biokompatiblen Membran mit langsamer Resorption, vorzugsweise zur gezielten Geweberegeneration, dadurch gekennzeichnet, daß die Membran entweder vernetztes Kollagen, ausgehend von einem Ausgangskollagen im koagulierten Zustand nach einer Koagulation eines Kollagengels mit einem Koagulierungsmittel, umfaßt, oder in Form einer Mischmembran vorliegt, die einen Schwamm einer Kollagen- oder Atelokollagen-Glykosaminoglykan-Mischung umfaßt, auf den ein Kollagengel vor der Vernetzung des Ganzen gegossen wurde, oder einen Schwamm einer Kollagen- und Chondroitin-4-sulfat-Mischung umfaßt, der unter einem Druck von 150 bar komprimiert wurde.

16. Verwendung nach Anspruch 15, dadurch gekennzeichnet, daß die Membran wie in einem der Ansprüche 2 bis 11 definiert ist.

17. Verwendung nach Anspruch 15 oder 16, dadurch gekennzeichnet, daß die Membran als Material zur gezielten Geweberegeneration in der Zahnchirurgie verwendet wird, um beispielsweise das Füllen von Zahnfleischtaschen, das Erhöhen maxillomandibularer Cristae und eine Periimplantat-Knochenregeneration zu ermöglichen.
